Europäisches Patentamt

European Patent Office

Office européen des brevets

(19)

(11) Numéro de publication: **0 253 941**
**B1**

(12) **FASCICULE DE BREVET EUROPEEN**

(45) Date de publication du fascicule du brevet:
**10.10.90**

(51) Int. Cl.⁵: **A61F 2/34**

(21) Numéro de dépôt: **86830197.9**

(22) Date de dépôt: **09.07.86**

(54) **Arthro-prothèse portante avec élément amortisseur.**

(43) Date de publication de la demande:
**27.01.88 Bulletin 88/4**

(45) Mention de la délivrance du brevet:
**10.10.90 Bulletin 90/41**

(84) Etats contractants désignés:
**AT BE CH DE FR GB IT LI LU NL SE**

(56) Documents cités:
**FR-A- 2 169 954**
**FR-A- 2 239 980**
**FR-A- 2 242 065**
**GB-A- 2 126 096**

(73) Titulaire: **CHENIL CONT S.p.A., Via Baldanzese, 247,
I-50041 Calenzano(IT)**

(72) Inventeur: **Sostegni, Giuliano, Via Cocchi, 14,
I-50019 Sesto Fiorentino§Firenze(IT)**

(74) Mandataire: **Martini, Lazzaro, Ufficio Brevetti Ing.
Lazzaro Martini Via dei Rustici 5, I-50122 Firenze(IT)**

## Description

La présente invention concerne une prothese selon le preambule de la revendication 1.

Il est connu que dans la phase dynamique la plus simple comme la marche lente, l'effort qu' une prothèse portante, en particulier de la hanche, doit supporter, atteint et dépasse une charge égale à au moins cinq fois le poids du patient. Dans de telles conditions, au moins deux problèmes importants se présentent dans les implantations de prothèses de la hanche qui sont la fixité de l'implantation dans l'interface prothèse - os et l'amortissement de la charge dans l'articulation.

La fixité de l'implantation peut désormais être considérée comme positivement résolue, dans les prothèses à implantation directe comme dans celles à implantation au moyen du ciment; au contraire l'amortissement de la charge dans l'articulation a effectué bien peu de progrès de sorte que les efforts transmis de la tête au cotyle de la prothèse sont d'intensité croissante, pratiquement à pic, pendant l'appui sur un seul pied et ceci sans ou à peu près sans déformation du joint de l'articulation.

Les premières tentatives faites pour résoudre ce problème consistent à interposer entre la tête fémorale et le cotyle d'une prothèse de la hanche, un tampon de matière plastique biocompatible, entourant complètement la tête de la prothèse et avec la surface concave et la surface convexe, lisses et d'épaisseur constante en tout point du tampon; les résultats obtenus ont été tout à fait négatifs en raison de la rigidité constante et très élévée du tampon, même sous de faibles charges, due à la non compressibilité de la matière utilisée.

En particuliér on connaît par le document FR-A 2 242 065 une prothèse coxo-fémorale se maintenant d'elle même comprenant une couvette metallique de l'articulation de la hanche à installer dans l'os du bassin, une calotte de cecéramique placée sur la tête fémorale et une enveloppe pleine en matière synthétique interposée pour compenser la surface d'articulation en ceramique. Cette enveloppe est fixée d'une part à la couvette et d'autre part à la calotte. Mais cette enveloppe étant toute pleine et fixe ne permet pas une réduction de son épaisseur et donc ne permet pas d'amortir efficacement les efforts dûs à la deambulation.

Plus récemment, on a adopté des gaines de même matière plastique, entourant partiellement la tête de la prothèse et pourvues de trous traversants, ou autrement avec la surface convexe pourvue de canaux répartis suivant les méridiens et les parallèles de la gaine et communicants entre eux destinés à diminuer de capacité avec l'écrasement de la gaine sous charge. Même ces types connus de gaines présentent une déformation trop limitée dans la direction de la charge, de sorte qu'ils ne résolvent pas la fonction d'amortissement des chocs qui se produisent dans l'articulation de la prothèse portante pendant la déambulation et en particulier pour de faibles charges.

Les essais de simulation effectuées ont mis en évidence ce fait et la confirmation est donnée par le fait que, après une cèrtaine période de temps, la gaine présente une concavité due à l'usure dans la zone d'effort maximum.

La présente invention a pour but d'améliorer sensiblement l'élasticité du joint d'articulation des prothèses portantes en permettant d'amortir plus efficacement les efforts dûs à la déambulation, en particulier ceux de valeurs les plus basses.

On est parvenu à ce résultat en conformité avec l'invention en adoptant l'idée de réaliser un berceau en plastique biocompatible non compressible, interposer entre la tête fémorale et le cotyle d'une prothèse portante, et dont la rigidité est progressivement croissante sous les charges impulsionnelles transmises par l'articulation, ledit berceau étant caractérisé en ce qu' il a la surface intérieure à gradins avec un premier gradin cylindrique, faisant saillie de la zone de fond du berceau et de hauteur telle à rester toujours en contact avec la zone correspondante de la tête de la prothèse, et avec d'autres gradins périfériques continus, espacès entre eux, parallèles et coaxiaux audit premier gradin et dont la hauteur est inférieure à celle du premier gradin pour permettre de venir en contact - sous des efforts croissants - avec des zones correspondantes de la tête de la prothèse de manière successive du fond vers la base du berceau.

Les avantages obtenus grace à la présente invention consistent essentiellement dans le fait qu' un amortisseur pour arthro-prothèse suivant l'invention présente une faible rigidité (rapport entre effort appliqué et déformation obtenue) pour des charges faibles de compression; que sa rigidité croît progressivement, d'abord lentement puis plus rapidement, avec l'accroissement de l'effort; qu' il est possible de faire varier la position du cotyle pour orienter l'axe du berceau suivant la direction d'effort maximum dans l'articulation; qu' il est possible de régler l'amplitude axiale de la déformation; qu' elle présent un couple de frottement et donc également une usure, très inférieur par rapport aux gaines pour arthro-prothèse utilisées aujourd' hui; qu' elle a un profil simple, de réalisation facile, dans des mesures différentes pour s'adapter à celles anatomiques des patients.

L'invention est exposée plus en détail par la suite à l'aide des dessins qui en représentent un exemple de réalisation.

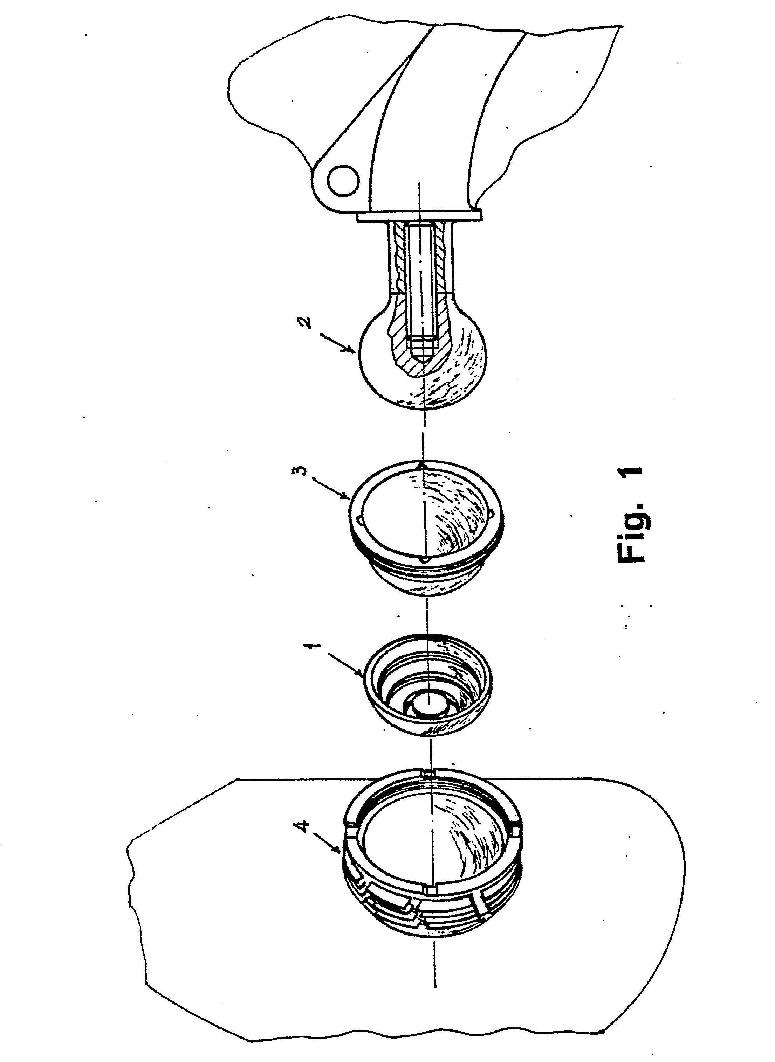

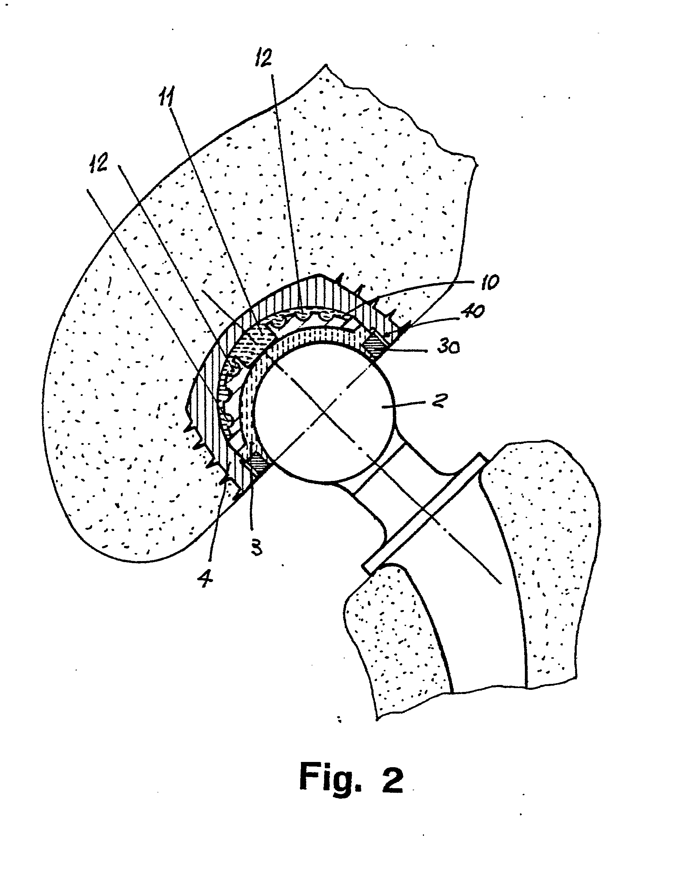

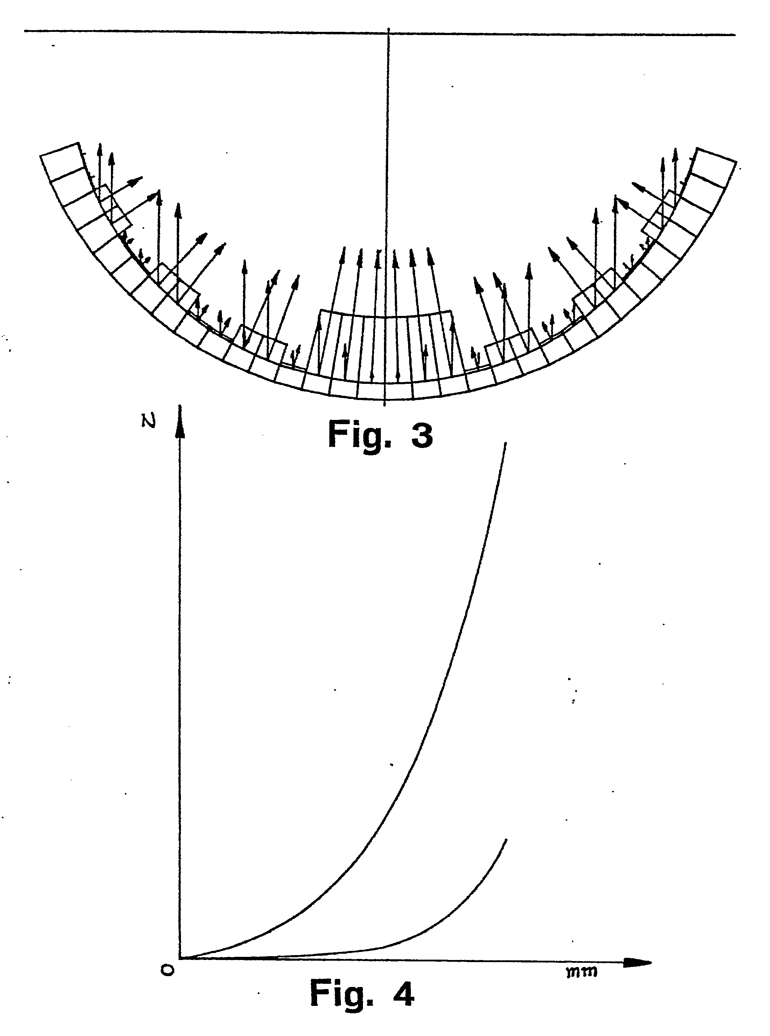

La figure 1 représente, en perspective éclatée, une prothèse d'hanche comportant un élément amortisseur selon l'invention; la figure 2 représente la section diamétrale de la prothèse de la figure 1 implantée; la figure 3 représente la répartition des réactions le long d'un arc concentrique d'un berceau pour prothèse de la hanche selon l'invention dans une forme pratique de réalisation; la figure 4 représente le diagramme efforts/déformations (N/mm) du berceau pour prothèse de la hanche de la figure 3 en comparaison à une gaine classique.

Les figures 1 et 2 montrent un berceau 1 pour prothèse de hanche totale, c'est à dire comprenant une tête fémorale métallique 2, un élément presseur en polyéthylène 3, à placer sur ladite tête 2, et un cotyle métallique 4 à fixer sur l'acétabule du patient. Ledit berceau 1 est constitué par un corps à forme de zone sphérique avec une base, dont la hauteur est à peine inférieure au rayon extérieur du presseur 3, et avec une surface externe, laquelle est destinée à se loger dans la cavité correspondante du cotyle 4, lisse. La section du berceau 1 suivant un plan passant par un méridien, est à gradins et ceci avec une paroi fine 10, un premier gradin central 11, c'est à dire saillant à partir de ladite paroi en correspondance du fond du berceau, en forme de cylindre circulaire et avec des gradins supplémentaires 12 périfériques, espacés, parallèles et coaxiaux au gradin central 11.

Lesdits gradins périfériques 12 ont un développement radial identique et sont espacés entre eux d'une distance supérieure à leur largeur; ledit gradin central 11 présente une hauteur sensiblement supérieure à celle des autres gradins 12 et une surface d'appui qui est d'environ 1/8 à 1/16 de la surface active de l'élément presseur 3; de cette manière, avec la prothèse au repos, seul le gradin 11 se trouve en contact avec la zone correspondante du presseur (visible sur la figure 2). Avec la prothèse soumise à un effort, mais pour des valeurs faibles de ce dernier, seulement le gradin central 11 est soumis à la compression et en subissant des incréments de déformation importants, comporte un amortissement élevé de la charge sur l'articulatin. Pour des valeurs plus élevées de l'effort, l'élément presseur 3 vient en contact avec des gradins périfériques 12, de manière successive de celui le plus proche à celui le plus loin du fond du berceau 1,ce qui détermine une augmentation de la rigidité du berceau de laquelle résultent de petits incréments de déformation.

Un embout à vis 30 qui guide l'élément presseur 3, en s'engageant dans un filetage correspondant 40 du cotyle 4, permet avantageusement de modifier de mamière préventive la distance du presseur 3 au berceau 1 et ainsi d'effectuer un réglage fin de la rigidité du berceau en fonction du poids du patient.

Il va de soi que le berceau 1 est déposé dans le cotyle 4 coaxialement à celui-ci et que le cotyle 4 doit être implanté dans l'acétabule incliné de manière opportune pour que son axe coincide avec la direction de l'effort maximum sur la prothèse.

Conformément à l'invention, il est prévu que les gradins, central 11 et périfériques 12, soient développés sur la surface convexe, au lieu de celle convace, du berceau ou également sur les deux surfaces, convace et convexe, du berceau.

Exemple. Par la suite, sont indiquées les valeurs géométriques des composantes principales d'une prothèse de hanche munie d'un amortisseur selon l'invention dans une forme considérée comme caractéristique pour une mise en oeuvre:

Presseur :        R = 19 mm
Berceau :         R = 22 mm
-Déformation   max = 4 mm
-Gradin central   H = 5 mm
                - - - o = 10 mm
Berceau : Gradins périfériques : 3 avec section semicirculaire
                - - - H = 2 mm

La figure 3 des dessins annexés met en évidence la répartition des forces radiales et des forces parallèles la direction de déformation par compression dudit berceau pendant l'exercice simulé avec des charges physiologiques (environ 100 Kg).

La courbe en bas du diagramme de la figure 4 raprésent la variation des déformations jusqu'à 4 mm du gradin central de ce berceau avec des charges physiologiques simulées; la courbe du haut du même diagramme raprésente la variation des déformations avec les mêmes charges physiologiques simulées, d'une gaine classique pour prothèse de hanche de dimensions identiques.

Comme on le voit, avec le berceau amortissant pour prothèse portante selon l' invention, pour des charges plus physiologiques de l'articulation il y a de grands incréments de déformation avec des charges faibles (environ 100 Kg) et, au contraire, de petits incréments pour des charges plus élevées (plus de 100 Kg).

Ceci confirme que le berceau, avant de transmettre des sollicitations élevées, se déforme de manière sensible dans sa partie moins résistante (c'est à dire le gradin central).

Des essais simulés ont permis d'affirmer que, à l'arrêt de l'effort sur l'articulation, le berceau réagissait également avec un module d'élasticité variable, contrairement aux gaines traditionelles, et plus précisément suivant la fonction

$$\frac{3,45 \ L/\triangle \ L}{Ex = e;z}$$

la courbe correspondante N/mm de décharge est décalée vers la droite par rapport à celle de charge, l'aire comprise entre les deux courbes représentant la faible dispersion d'énergie par hystérésis, laquelle par ailleurs ne fait pas prévoir de problème de réchauffement excessif du bercau.

**Revendications**

1) Prothèse comprenant une tête (2), un élément presseur (3) lié à la tête, un cotyle (4) et un amortisseur interposé entre l'élément presseur et le cotyle, l'amortisseur étant constitué d'un berceau (1) en forme de portion sphérique à une base, destinée, d'un côté, à mettre en place l'élément presseur (3), et de l'autre côté, à se loger dans la cavité du cotyle (4), caractérisé en ce que ledit berceau (1) possède une section dans un plan méridien, à gradins avec un paroi fine (10), un premier gradin (11) centrale, cylindrique, saillant à partir de la zone polaire de la surface concave ou convexe de la paroi (10) du berceau et au moins un second gradin (12) périphérique, saillant à partir du même côté que le premier gradin (11) par rapport à la paroi (10) du berceau et espacé, parallèle et coaxial au dit premier gradin (11).

2) Amortisseur pour arthro-prothèse portante selon la revendication 1), caractérisé en ce que ledit berceau (1) a une hauteur inférieure à celle de l'élément presseur, tête correspondant (3,2) de prothèse auquel il est destiné.

3) Amortisseur pour arthro-prothèse portante selon la revendication 1), caractérisé en ce que ledit gradin central (11) du berceau (1) est de hauteur supérieure à celle des gradins périphériques (12) et pos-

sède une base active comportant une surface comprise entre 1/8 et 1/16 de la surface active de l'élément presseur,tête correspondant (3,2) de prothèse auquel il est destiné pour obtenir une rigidité du berceau variable sous les efforts de compression de l'articulation et avec l'incrément maximum de déformation pour les valeurs les plus basses de l'effort.

4) Amortisseur pour arthro-prothèse portante selon la revendication 1), caractérisé en ce que les gradins (12) du berceau (1) sont de hauteur égale en direction radiale.

5) Amortisseur pour arthro-prothèse portante selon la revendication 1), caractérisé en ce que chaque gradin périférique (12) a une section semicirculaire.

6) Amortisseur pour arthro-prothèse portante selon la revendication 1), caractérisé en ce que les gradins périfériques (12) sont espacés entre eux d'une distance supérieure leur propre hauteur.

7) Amortisseur pour arthro-prothèse portante selon la revendication 1), caractérisé en ce que la tolérance de l'accouplement du berceau (1) avec l'élément presseur (3) de la prothèse est réglée au moyen d'un embout vis (30) lequel guide axialement l'élément presseur (3) et s'engage dans un filetage correspondant (40) du cotyle (4).

**Patentansprüche**

1. Prothese mit einem Kopf (2), einem mit dem Kopf verbundenen Druckelement (3), einer Gelenkpfanne (4) und einem Puffer zwischen dem Druckelement und der Gelenkpfanne, wobei der Puffer aus einem Käfig (1) in Form eines Kugelabschnittes als Basis besteht, der einerseits zur Lageanordnung des Druckelementes (3) und andererseits dazu dient, in der Ausnehmung der Gelenkpfanne (4) gelagert zu werden, dadurch gekennzeichnet, daß der Käfig (1) in einer Meridianebene einen Stufenabschnitt mit einer dünnen Wand (10) besitzt, wobei eine erste, mittlere, zylindrische Stufe (11) von der Polzone der konkaven oder konvexen Fläche der Wand (10) des Käfigs hervorragt und wenigstens eine zweite, periphere Stufe (12) von derselben Seite wie die erste Stufe (11) gegenüber der Wand (10) des Käfigs ausgeht und zur ersten Stufe (11) parallel und Koaxial getrennt ist.

2. Puffer für eine tragende Gelenksprothese nach Anspruch 1, dadurch gekennzeichnet, daß der Käfig (1) eine Höhe hat, die geringer als jene, dem Druckelement entsprechenden Kopfes (3, 2) der Prothese, für die er bestimmt ist, ist.

3. Puffer für eine tragende Gelenksprothese nach Anspruch 1, dadurch gekennzeichnet, daß die mittlere Stufe (11) des Käfigs (1) eine Höhe hat, die größer ist als jene der Randstufen (12) und eine wirksame Basis mit einer Fläche zwischen 1/8 und 1/16 der wirksamen Fläche des dem Druckelement entsprechenden Kopfes (3, 2) der Prothese hat, für die er bestimmt ist, um unter den Druckkräften der Gelenksbewegung eine veränderliche Steifigkeit des Käfigs und mit einem maximalen Verformungsschritt für die tiefsten Werte der Kraft zu erreichen.

4. Puffer für eine tragende Gelenksprothese nach Anspruch 1, dadurch gekennzeichnet, daß die Stufen (12) des Käfigs (1) in radialer Richtung gleiche Höhe haben.

5. Puffer für eine tragende Gelenksprothese nach Anspruch 1, dadurch gekennzeichnet, daß jede Randstufe (12) halbkreisförmig ist.

6. Puffer für eine tragende Gelenksprothese nach Anspruch 1, dadurch gekennzeichnet, daß die Randstufen (12) untereinander um einen Abstand getrennt sind, der größer als ihre eigene Höhe ist.

7. Puffer für eine tragende Gelenksprothese nach Anspruch 1, dadurch gekennzeichnet, daß die Toleranz der Kupplung des Käfigs (1) mit dem Druckelement (3) der Prothese mit Hilfe eines Schraubringes (30) eingestellt wird, der das Druckelement (3) axial führt und in eine entsprechende Verschraubung (40) der Gelenkpfanne (4) eingreift.

**Claims**

1. Prosthesis (false teeth) containing one head (2), a pressing element (3) connected to the head, a cup (4) and a shock absorber interposed between the pressing element and the cup, the shock absorber consisting of a cradle in the form of the spherical part on a base designed, on the one side, to place the pressing element (3) and on the other side to position itself in the cavity of the cup (4), characterized by the fact that the said cradle (1) has a section in a meridian plane which is stepped with a thin wall (10), a first central cylindrical step (11) projecting from the polar zone of the concave or convex surface of the wall (10) of the cradle and at least one second peripheral step (12) projecting from the same side as the first step (11) relative to the wall (10) of the cradle and spaced, parallel and coaxially from the said first step (11).

2. Prosthesis joint shock absorber according to claim 1, characterized by the fact that the said cradle (1) has a lower height than the pressing element corresponding head (3, 2) of the prosthesis for which it is intended.

3. Prosthesis joint shock absorber according to claim 1, characterized by the fact that the said central step (11) of the cradle (1) is of a greater height than the peripheral steps (12) and has an active base consisting of a surface comprising of between 1/8 and 1/16 of the active surface of the pressing element corresponding head (3, 2) of the prosthesis for which it is intended to obtain a variable rigidity of the cradle under the forces of compression of the articulation and with the maximum increase of deformation for the lowest values of force.

4. Prosthesis joint shock absorber according to claim 1, characterized by the fact that the steps (12) of the cradle (1) are of equal height in the radial direction.

5. Prosthesis joint absorber according to claim 1, characterized by the fact that each peripheral step (12) has a semicircular section.

6. Prosthesis joint shock absorber according to claim 1, characterized by the fact that the peripheral steps (12) are spaced out by a distance greater than their own height.

7. Prosthesis joint shock absorber according to claim 1, characterized by the fact that the coupling of the cradle (1) to the pressing element (3) of the prosthesis is controlled by means of a screwed joint (30) which guides the pressing element (3) axially and engages in a corresponding thread (40) of the cup (4).

Fig. 1

**Fig. 2**

**Fig. 3**

**Fig. 4**